# FASCICULE DE BREVET EUROPEEN

(11) **EP 1 206 240 B1**
(45) Date de publication et mention de la délivrance du brevet: **20.10.2004**
(21) Numéro de dépôt: 00958748.6
(22) Date de dépôt: 25.08.2000
(51) Int. Cl.: A61K 7/46

(54) **COMPOSITION NON ETHANOLIQUE COMPRENANT UN HYDROFLUOROETHER**
ETHYLALKOHOL-FREIE ZUSAMMENSETZUNG DIE EINEN HYDROFLUOROETHER ENTHALT
NON-ETHANOL COMPOSITION COMPRISING HYDROFLUOROETHER

(30) Priorité: 26.08.1999 FR 9910821
(43) Date de publication de la demande: 22.05.2002
(73) Titulaire: LVMH RECHERCHE, 45800 St. Jean de Braye (FR)
(72) Inventeur: SOUVIE, Marie-Laure, F-45000 Orléans (FR); BATON, Gérard, F-28000 Chartres (FR)
(74) Mandataire: Portal, Gérard
(86) Numéro de dépôt international: PCT/FR2000/002384
(87) Numéro de publication internationale: WO 2001/013875

(56) Documents cités:
- EP-A- 0 845 523
- EP-A- 1 029 527
- WO-A-94/10970
- WO-A-99/26600

## Description

L'invention concerne essentiellement une composition non éthanolique comprenant un hydrofluoroéther perfluoré et au moins un co-solvant autre que l'eau et l'éthanol comprenant un ester d'un polyacide et son utilisation dans des compositions parfumantes.

De telles compositions parfumantes sont de préférence des compositions de parfum sans éthanol dont tous les composants sont miscibles entre eux donnant ainsi à la composition l'aspect d'un liquide limpide.

Dans les produits parfumants (parfum, eau de toilette, etc.), la présence d'alcool (éthanol), qui est utilisé principalement comme agent solubilisant du concentré de parfum, pose un certain nombre de problèmes bien connus à l'homme de l'art. C'est pourquoi il a été recherché depuis de nombreuses années des produits parfumants évitant l'apport d'alcool en le remplaçant par d'autres agents solubilisants.

A titre d'exemples, on pourra se reporter aux documents WO 99/18925 et US 5,468,725, qui décrivent des compositions de parfum sans alcool en utilisant respectivement des silicones comme agent solubilisant et la technique des micro-émulsions.

L'un des problèmes généraux qui se pose est la préservation olfactive de la composition parfumée du concentré et notamment la neutralité olfactive des agents de solubilisation utilisés.

Par ailleurs, il est aussi connu par le document WO 99/11225 des préparations cosmétiques contenant comme ingrédient essentiel au moins 1 % d'hydrofluoroéther dans le but d'améliorer la tolérance de ces compositions vis-à-vis de la peau et d'améliorer le toucher du produit cosmétique.

Il est également connu par le document WO 99/26600 l'utilisation d'hydrofluoroéthers perfluorés comme agents de dissolution de composés aromatiques pour la réalisation d'une composition cosmétique. Dans ce document, il est envisagé d'une. manière générale en page 3, lignes 21 à 25, la possibilité d'ajouter au moins un co-solvant qui est indiqué comme étant préférentiellement choisi dans le groupe constitué par l'éthanol et l'eau, c'est-à-dire en pratique un mélange hydroalcoolique. Les exemples qui sont donnés dans ce document concernent tous l'utilisation exclusive d'un hydroperfluoroéther pour solubiliser des huiles essentielles. Les hydroperfluoroéthers cités sont le méthoxynonafluorobutane, en abrégé MNFB, exemples 1 et 2, l'éthoxynonafluorobutane, en abrégé ENFB, exemples 3 à 8, et également le propoxy-undécafluoropentane.

Dans le cadre de compositions parfumantes, il existe un problème spécifique qui consiste dans la solubilité nécessaire de tous les constituants d'une telle composition parfumante. Un composant principal est constitué par un concentré de parfum qui contient des huiles essentielles ainsi que divers composants tels que des agents émulsionnants ou tensioactifs, des esters gras, ou des dérivés de cellulose, ainsi que d'autres composants qui sont bien connus de l'homme de l'art.

Dans le cadre d'essais réalisés en vue d'obtenir des compositions sans éthanol ou alcool, le déposant a réalisé des essais tendant à vérifier si des hydrofluoroéthers perfluorés pouvaient être seuls capables de réaliser une solubilisation totale de tels concentrés de parfum en vue de la préparation de compositions parfumantes, ces concentrés de parfum étant disponibles dans le commerce et commercialisés par des sociétés spécialisées dans leur fabrication telles que la société GIVAUDAN, Suisse, la société FIRMENICH, Suisse ou la société International Flavors & Fragrances (IFF), USA. Or, il s'avère que les essais ont tous été négatifs, en ce sens que les liquides obtenus n'étaient pas limpides, faisant apparaître clairement deux phases distinctes.

Ainsi, la présente invention a pour but principal de résoudre le nouveau problème technique consistant en la fourniture d'une solution qui permette de réaliser des compositions capables d'obtenir la solubilisation complète pour aboutir à un liquide essentiellement limpide de concentrés de parfum en vue notamment de la préparation de compositions parfumantes.

La présente invention a encore pour but principal de résoudre le nouveau problème technique consistant en la fourniture d'une solution qui permette de réaliser la solubilisation précitée de concentrés de parfum à l'aide d'agents solubilisants qui soient neutres ou sensiblement neutres vis-à-vis des qualités olfactives dudit concentré de parfum, en vue de la préparation notamment de compositions parfumantes d'excellente qualité.

La présente invention a encore pour but principal de résoudre les deux nouveaux problèmes techniques énoncés ci-dessus, selon une solution qui soit sans éthanol ou alcool et qui n'utilise pas comme agent co-solvant l'eau, l'éthanol ou leurs mélanges.

L'invention permet de résoudre l'ensemble de ces problèmes techniques pour la première fois d'une manière satisfaisante, simple, utilisable à l'échelle industrielle et cosmétique notamment pour la mise au point de compositions parfumantes sans éthanol ou alcool, d'excellente qualité, en particulier des parfums et eaux de toilette.

Ainsi, selon un premier aspect, la présente invention fournit une composition sans éthanol ou alcool, comprenant un hydrofluoroéther, caractérisée en ce qu'elle comprend au moins un ester d'un polyacide.

Il a été découvert, d'une manière surprenante, que la combinaison d'un hydrofluoroéther et d'un ester de polyacide, agissant comme co-solvant, permettait de réaliser une solubilisation quasi-parfaite ou parfaite de concentrés de parfum, permettant d'obtenir une solution essentiellement limpide permettant de fabriquer des compositions parfumantes d'excellente qualité. En outre, il a été observé, également de manière surprenante, que la combinaison de l'hydrofluoroéther et de l'ester de polyacide constituait un agent solubilisant neutre ou essentiellement neutre d'un point de vue olfactif vis-à-vis des parfums concernés.

Le composant hydrofluoroéther est de préférence un composant perfluoré. Ces composés sont bien connus de l'homme de l'art et sont, par exemple, du type de ceux décrits dans le document WO 99/11225 et peuvent présenter la formule générale CₙHₘFₚ-O-CₓH_{y}F_{z} dans laquelle n est un nombre allant de 1 à 12 ; m est un nombre allant de 0 à 25 ; p est un nombre allant de 0 à 11 ; m + p = 2n + 1 ; x est un nombre de 1 à 12 ; y est un nombre de 0 à 25 ; x est un nombre de 0 à 11 et y + z = 2x + 1. Dans cette formule, il est exclu qu'à la fois m et y soient égaux à 0 et qu'à la fois p et z soient égaux à 0, comme décrit dans ce document pour assurer l'exactitude de la formule chimique.

Certains composés hydrofluoroéthers perfluorés, répondant à la formule générale ci-dessus, sont décrits dans le document WO 99/26600, à savoir le méthoxy-nonafluorobutane, l'éthoxy-nonafluorobutane, le propoxy-undécafluoropentane. On peut encore utiliser le méthoxy-heptafluoropropane disponible dans le commerce.

L'ester de polyacide précité est de préférence un ester de polyacide, hydroxylé ou non, qui est encore de préférence formé avec un alcool saturé ou non saturé, linéaire ou ramifié, ayant un nombre d'atomes de carbone compris entre 1 et 30, de préférence compris entre 1 et 12, en particulier éthanol, isopropanol, éthyl-2-hexanol.

Le polyacide présente de préférence un nombre d'atomes de carbone compris entre 3 et 10. La chaîne carbonée du polyacide peut être linéaire ou ramifiée, saturée ou insaturée, avec une ou plusieurs insaturations.

De plus, la chaîne carbonée des polyacides peut être substituée par un ou plusieurs groupements hydroxy, ou comporter une ou plusieurs fonctions cétone. Les groupements hydroxy précités peuvent être acétylés.

Les esters de polyacides utilisables pour mettre en oeuvre l'invention sont de préférence sensiblement non polaires.

Il peut s'agir d'esters partiels ou totaux du polyacide.

De préférence, toutes les fonctions acides du polyacide sont estérifiées.

De préférence, lorsqu'il s'agit de groupements à longues chaînes carbonées, ces chaînes sont ramifiées. Il en est ainsi du groupement 2-éthyl-hexyle qui comprend 8 atomes de carbone.

Les groupements esters préférés sont les groupements éthyle. iso-propyle, 2-éthyl-héxyle.

Les polyacides utilisables selon l'invention sont avantageusement choisis parmi :
→ les diacides saturés, tels que :
   - malonique
   - succinique
   - glutarique
   - adipique
   - pimélique
   - subérique
   - azélaïque
→ les diacides mono-insaturés, tels que :
   - fumarique
   - maléique
   - citraconique*
   - itaconique
   - mésaconique
→ les diacides diinsaturés, tel que :
   - muconique
→ les diacides mono-hydroxylés, tels que :
   - tartronique
   - malique
   - citramalique
→ les diacides dihydroxylés, tels que :
   - dihydroxy-maléique
   - tartrique
→ les diacides tétrahydroxylés, tels que :
   - dihydroxytartrique
   - galactarique
   - glucarique
→ les kéto diacides, tels que :
   - mésoxalique
   - oxalacétique
   - 2-oxoglutarique
   - 3-oxoglutarique
→ les dikéto diacides, tel que :
   - 2,3-dikétoadipique
→ les triacides saturés, tels que :
   - tricarballylique
   - citrique (triacide monohydroxylé)
→ les triacides insaturés, tel que :
   - aconitique

* (diacide ramifié)

Les polyacides préférés sont l'acide citrique et, plus particulièrement, l'acide adipique.

Les esters de l'acide citrique sont de préférence le tri-éthyl-citrate, le tri-(2-éthyl-héxyle) citrate et l'acétyl-triéthyl citrate. Ces esters sont disponibles dans le commerce.

Les esters préférés de l'acide adipique sont le diisopropyl adipate (souvent dénommé iso-adipate) et le di-(2-éthyl-hexyl) adipate. Ces esters sont également disponibles dans le commerce.

Les proportions relatives en poids de l'ester de polyacide relativement au composant hydrofluoroéther peuvent varier dans des limites qui ne modifient pas sensiblement le caractère olfactif des parfums. Généralement, l'ester de polyacide pourra représenter de 0,1 à 30 % en poids du composant hydrofluoroéther, et en particulier de 1 à 20 % en poids.

On peut également prévoir tout autre composant complémentaire dans ladite composition, en particulier un deuxième co-solvant, tel qu'un silicone ou un composant améliorant les qualités d'une composition parfumante, telle que la rémanence de ladite composition sur la peau, par exemple un phtalate tel que le diéthylphtalate. Cependant, le silicone est préféré car il combine à la fois les propriétés de co-solvant ou additif de solubilisation et de rémanence.

De préférence, la proportion en silicone sera calculée pour correspondre à environ 1 à 20 % en poids par rapport au poids de la composition parfumante finale.

A titre de silicones, on pourra utiliser un diméthicone ou un cyclométhicone, en particulier le pentacyclométhicone, disponible dans le commerce, ou un organotrisiloxane tel que par exemple décrit dans le document PCT publié sous le numéro WO 99/06018 et en particulier commercialisé sous le nom de SILATRIPHENE par la société RHODIA, France. Avantageusement, on pourra utiliser les silicones volatiles, en particulier des diméthicones commercialisées par la société américaine DOW CORNING telle que le DC200 Fluid 1 centiStokes, ou de préférence le DC200 Fluid 0,65 centiStokes.

Cette composition peut aussi comprendre différents additifs habituellement utilisés pour préparer des compositions de parfums ou d'eau de toilette, et par exemple , des filtres U-V, des agents anti-oxydants, des colorants, etc. De tels additifs complémentaires seront généralement ajoutés à une proportion pouvant aller jusqu'à 2 % en poids de la composition finale.

Selon un premier mode de réalisation préféré, l'invention fournit une composition sans éthanol ou alcool, comprenant un hydrofluoroéther, caractérisée en ce qu'elle comprend, en pourcentage en poids :
- hydrofluoroéther 65 à 85 %
- triester d'acide citrique (tel que le triéthyl citrate) 4 à 7 %
- 2^{ème} co-solvant ou solubilisant :
   silicone tel que DC200 Fluid de DOW CORNING, USA 8 à 16 %
- concentré de parfum 5 à 20 %

Selon un deuxième mode de réalisation préféré, l'invention fournit une composition sans éthanol ou alcool, comprenant un hydrofluoroéther, caractérisée en ce qu'elle comprend, en pourcentage en poids :
- hydrofluoroéther 65 à 85 %
- concentré de parfum 5 à 20 %
- iso-adipate (ou diisopropyladipate) 10 à 20 %.

Dans le cadre de l'invention, l'hydrofluoroéther sert généralement à constituer le solde de la composition, mais celle-ci peut éventuellement comprendre les différents additifs habituellement utilisés pour préparer des compositions de parfum ou d'eau de toilette citées ci-dessus.

Il a été observé, dans le cadre de l'invention, que les compositions utilisant comme 2^{ème} co-solvant ou additif de solubilisation l'iso-adipate présentent généralement un toucher moins gras que celui des compositions utilisant l'association ou combinaison de citrate et de silicone.

Ceci constitue un avantage technique important, inattendu pour l'homme de l'art, car le toucher des compositions à base d'iso-adipate est ainsi plus proche de celui des compositions parfumées alcooliques classiques, telles que les eaux de toilette et les parfums.

Selon un deuxième aspect, la présente invention couvre aussi l'utilisation d'un ester d'un polyacide tel que précédemment défini en combinaison avec un hydrofluoroéther comme agent solubilisant d'un concentré de parfum en vue notamment de la préparation de compositions parfumantes, telles que parfums ou eaux de toilette.

Selon un troisième aspect, la présente invention couvre aussi une composition parfumante, sans éthanol ou alcool, comprenant un concentré de parfum, un hydrofluoroéther, caractérisée en ce qu'elle comprend en outre au moins un ester d'un polyacide en quantité suffisante pour obtenir un aspect essentiellement limpide de ladite composition.

Cette composition parfumante avantageusement comprendra d'environ 65 à environ 85 % en poids de composant hydrofluoroéther par rapport au poids final de la composition parfumante.

Selon une autre caractéristique avantageuse de cette composition parfumante, celle-ci comprendra d'environ 1 à environ 20 % en poids d'ester de polyacide précité, de préférence de 3 à 20 % en poids, par rapport au poids final de la composition parfumante.

Selon une autre caractéristique avantageuse de la composition parfumante, celle-ci comprendra d'environ 5 à environ 20 % en poids de concentré de parfum. Dans ce cadre, dans le cas d'eau de toilette, la concentration en concentré de parfum sera généralement de l'ordre de 5 % en poids par rapport au poids final de l'eau de toilette. Dans le cas de parfum. la concentration en concentré de parfum sera généralement comprise entre 10 et 20 % en poids de la composition finale de parfum.

Ainsi, la composition parfumante sera de préférence constituée par une eau de toilette ou un parfum.

Naturellement, que ce soit pour le deuxième aspect ou le troisième aspect, diverses variantes de réalisation résultent du premier aspect. Ainsi, l'hydrofluoroéther et l'ester d'un polyacide sont tels que définis dans le cadre du premier aspect. Il en est de même pour tout autre composant complémentaire qui peut être présent et qui a été décrit dans le cadre du premier aspect.

Par ailleurs, dans le cadre de l'un quelconque des aspects de l'invention. la composition peut avantageusement contenir jusqu'à 2 % en poids de la composition finale de tout additif habituellement utilisé pour préparer des compositions parfumantes, tels que parfums ou eaux de toilette, par exemple des filtres UV, des agents anti-oxydants, des colorants, etc.

Le procédé de préparation de la composition est aisément compréhensible par un homme de l'art. Généralement, il comprendra tout d'abord l'ajout de l'ester de polyacide au concentré de parfum, puis l'ajout des éventuels composés complémentaires, et en particulier un deuxième co-solvant tel qu'un silicone ou un composant améliorant les qualités de la composition parfumante telle que la rémanence de la composition sur la peau, par exemple un phtalate précité, les éventuels autres additifs en particulier des filtres UV, des agents anti-oxydants ou des colorants, et enfin le composant hydrofluoroéther qui sera de préférence ajouté en dernier et qui pourra aussi généralement constituer le solde de la formule.

La présente invention sera maintenant illustrée à l'aide d'exemples de compositions de parfum et d'eau de toilette, sans éthanol ou alcool, d'aspect totalement limpide ou solution parfaite, donnés simplement à titre d'illustration et qui ne sauraient donc en aucune façon limiter la portée de l'invention. Dans les exemples, les pourcentages sont donnés en poids sauf indication contraire.

### Exemple 1 de composition de parfum sans éthanol ou alcool, selon l'invention

Cette composition de parfum présente la composition suivante :
- éthoxynonafluorobutane 68 %
- triéthylcitrate 8 %
- concentré de parfum disponible dans le commerce 20 %
- diéthylphtalate 4 %

Cette composition est préparée de la manière suivante ;

On ajoute tout d'abord le triéthylcitrate au concentré de parfum du commerce que l'on mélange intimement, puis le diéthylphtalate et enfin on ajoute l'éthoxynonafluorobutane.

On constate que cette composition de parfum ainsi préparée, sans éthanol ou alcool, présente un aspect totalement limpide ou de solution parfaite et que les qualités olfactives du concentré de parfum sont totalement préservées.

### Exemple 2 de composition de parfum sans éthanol ou alcool, selon l'invention

Cette composition de parfum présente les ingrédients suivants :
- méthoxynonafluorobutane 71 %
- trioctylcitrate 6 %
- silicone de chez DOW CORNING réf. DC200 Fluid 0,65cs 8%
- concentré de parfum du commerce 15 %

Cette composition est préparée de manière similaire à celle de l'exemple 1. Le silicone étant ajouté après le citrate.

On constate également le caractère totalement limpide ou de solution parfaite de cette composition et la préservation totale des qualités olfactives des parfums.

### Exemple 3 de composition de parfum sans éthanol ou alcool, selon l'invention

Cette composition de parfum présente les ingrédients suivants :
- méthoxy heptafluoropropane 75 %
- triéthylcitrate 6 %
- mélange 50:50 en poids de diéthylphtalate/silicone DC200 Fluid 1cs de DOW CORNING 9 %
- concentré de parfum du commerce 10 %

Le mélange est réalisé de manière similaire aux exemples 1 et 2 pour obtenir également une composition de parfum sans éthanol ou alcool, aspect totalement limpide ou de solution parfaite, préservant les capacités olfactives des parfums.

### Exemple 4 : Composition d'eau de toilette sans éthanol ou alcool, selon l'invention

Cette composition d'eau de toilette présente les ingrédients suivants :
- méthoxy-nonafluorobutane 82 %
- triéthylcitrate 3 %
- pentacyclométhicone 10 %
- concentré de parfums du commerce 5 %.

Cette composition est préparée selon la même procédure de mélange que celle décrite aux exemples précédentes.

### Exemple 5 : Composition de parfum sans éthanol ou alcool, selon l'invention

Cette composition de parfum présente les ingrédients suivants, encore en pourcentage en poids :
- concentré de parfum du commerce environ 10
- éthoxynonafluorobutane environ 77
- diisopropyladipate environ 13

Cette composition utilisant comme ester co-solvant le diisopropyladipate présente généralement un toucher moins gras que celui des compositions utilisant l'association de citrate et de silicone ce qui permet d'aboutir à l'avantage technique important, inattendu que le toucher de cette composition est plus proche de celui des compositions parfumées alcooliques classiques, telles que les eaux de toilette.

D'autres variantes de réalisation de ces exemples sont bien connues de l'homme de l'art et peuvent inclure par exemple l'incorporation de divers autres additifs habituellement utilisés pour préparer des compositions parfumantes, tels que parfums ou eaux de toilette, et par exemple des filtres UV, des agents anti-oxydants, des colorants, etc. De tels additifs complémentaires seront généralement ajoutés à une proportion pouvant aller jusqu'à 2 % en poids de la composition finale.

## Revendications

1. Composition sans éthanol ou alcool comprenant un hydrofluoroéther, **caractérisée en ce qu'**elle comprend au moins un ester d'un polyacide.

2. Composition selon la revendication 1, **caractérisée en ce que** le composant hydrofluoroéther est un composant perfluoré, en particulier de formule générale CₙHₘFₚ-O-CₓH_{y}F_{z} dans laquelle n est un nombre allant de 1 à 12 ; m est un nombre allant de 0 à 25 ; p est un nombre allant de 0 à 11 ; m + p = 2n + 1 ; x est un nombre de 1 à 12 ; y est un nombre de 0 à 25 ; x est un nombre de 0 à 11 et y + z = 2x + 1, dans cette formule, il est exclu qu'à la fois m et y soient égaux à 0 et qu'à la fois p et z soient égaux à 0.

3. Composition selon la revendication 1 ou 2, **caractérisée en ce que** le composant hydrofluoroéther est choisi parmi le méthoxynonafluorobutane, l'éthoxy-nonafluorobutane, le propoxy-undécafluoropentane et le méthoxy-heptafluoropropane.

4. Composition selon l'une des revendications précédentes, **caractérisée en ce que** l'ester de polyacide précité est un ester de polyacide, hydroxylé ou non, de préférence formé avec un alcool, saturé ou insaturé, linéaire ou ramifié, ayant un nombre d'atomes de carbone compris entre 1 et 30, de préférence compris entre 1 et 12, en particulier éthanol, isopropanol ou 2-éthylhexanol.

5. Composition selon l'une quelconque des revendications précédentes, **caractérisée en ce que** le polyacide présente un nombre d'atomes de carbone compris entre 3 et 10, et comprend une chaîne carbonée linéaire ou ramifiée, saturée ou insaturée, ladite chaîne carbonée pouvant être substituée par un ou plusieurs groupements hydroxy, ou comporter une ou plusieurs fonctions cétones, lesdits groupements hydroxy pouvant être acétylés.

6. Composition selon l'une quelconque des revendications précédentes, **caractérisée en ce que** le polyacide est totalement estérifié par un alcool tel que défini à la revendication 4.

7. Composition selon l'une quelconque des revendications précédentes, **caractérisée en ce que** le polyacide est un diacide saturé tel que malonique, succinique, glutarique, adipique, pimélique, subérique, azélaïque, un diacide mono-insaturé, tel que fumarique, maléique, citraconique, itaconique, mésaconique, un diacide diinsaturé tel que muconique, un diacide monohydroxylé tel que tartronique, malique, citramalique, un diacide dihydroxylé tel que dihydroxy-maléique, tartrique, un diacide tétrahydroxylé tel que dihydroxytartrique, galactarique, glucarique, un kéto diacide tel que mésoxalique, oxalacétique, 2-oxoglutarique, 3-oxoglutarique, un dikéto diacide tel que 2,3-dikétoadipique, un triacide saturé tel que tricarballylique, citrique ou un triacide insaturé tel que aconitique.

8. Composition selon la revendication 7, **caractérisée en ce que** le polyacide est l'acide citrique ou l'acide adipique.

9. Composition selon l'une quelconque des revendications précédentes, **caractérisée en ce que** l'ester du polyacide est un ester sensiblement non polaire.

10. Composition selon l'une quelconque des revendications précédentes, **caractérisée en ce que** l'ester de polyacide précité est choisi parmi le groupe constitué par le triéthylcitrate, le tri-(2-éthyl-hexyl) citrate, le diisopropyladipate et le di-(2-éthyl-hexyl) adipate.

11. Composition selon l'une quelconque des revendications précédentes, **caractérisé en ce que** l'ester de polyacide précité représente 0,1 à 30 % en poids du composant hydrofluoroéther, en particulier de 1 à 20 % en poids.

12. Composition selon l'une quelconque des revendications précédentes, **caractérisée en ce qu'**elle comprend un composant complémentaire, en particulier un deuxième co-solvant, tel qu'un silicone, ou un composant améliorant les qualités d'une composition parfumante, telle que la rémanence de ladite composition sur la peau, par exemple un phtalate, tel que le diéthylphtalate.

13. Composition selon l'une quelconque des revendications précédentes, **caractérisée en ce qu'**elle comprend un silicone, en particulier choisi parmi un silicone volatile, un diméthicone, un cyclométhicone, en particulier le pentacyclométhicone, ou un organo-trisiloxane, de préférence le silicone représente de 1 à 20 % en poids de la composition.

14. Utilisation d'un ester d'un polyacide tel que défini à l'une quelconque des revendications précédentes, avec un hydrofluoroéther, comme agent solubilisant d'un concentré de parfums, en vue notamment de la préparation d'une composition parfumante, tels que parfums, ou eaux de toilette.

15. Composition parfumante sans éthanol ou alcool comprenant un concentré de parfums, un hydrofluoroéther, **caractérisée en ce qu'**elle comprend en outre au moins un ester d'un polyacide en quantité suffisante pour obtenir un aspect essentiellement limpide de ladite composition.

16. Composition parfumante selon la revendication 15, **caractérisée en ce que** le composant hydrofluoroéther est présent en une quantité d'environ 65 à environ 85 % en poids par rapport au poids final de la composition parfumante.

17. Composition selon la revendication 15 ou 16, **caractérisée en ce qu'**elle comprend d'environ 1 à environ 20 % en poids d'ester de polyacide, de préférence de 3 à 20 % en poids par rapport au poids final de la composition parfumante.

18. Composition selon l'une quelconque des revendications 15 à 17, **caractérisée en ce qu'**elle comprend d'environ 5 à environ 20 % en poids de concentré de parfums.

19. Composition selon l'une quelconque des revendications 15 à 18, **caractérisée en ce qu'**il s'agit d'une eau de toilette comprenant une concentration en concentré de parfums de l'ordre de 5 % en poids par rapport au poids final de l'eau de toilette.

20. Composition selon l'une quelconque des revendications 15 à 19, **caractérisée en ce qu'**il s'agit d'un parfum, la concentration en concentré de parfums étant comprise entre 10 et 20 % en poids de la composition finale de parfums.

21. Composition selon l'une quelconque des revendications 15 à 20, **caractérisée en ce que** l'hydrofluoroéther et/ou l'ester de polyacide est tel que défini à l'une quelconque des revendications 1 à 11.

22. Composition selon l'une quelconque des revendications 15 à 21, **caractérisée en ce qu'**elle comprend un composant complémentaire, en particulier un deuxième co-solvant, tel qu'un silicone, ou un composant améliorant les qualités d'une composition parfumante, telle que la rémanence de ladite composition sur la peau, par exemple un phtalate, tel que le diéthylphtalate.

23. Composition selon l'une quelconque des revendications 15 à 22, **caractérisée en ce qu'**elle comprend jusqu'à 2 % en poids de la composition finale d'au moins un additif complémentaire, par exemple un filtre UV , un agent antioxydant, un colorant.

## Patentansprüche

1. Zusammensetzung ohne Ethanol oder Alkohol, die einen Hydrofluorether enthält, **dadurch gekennzeichnet, daß** sie mindestens einen Polysäureester enthält.

2. Zusammensetzung nach Anspruch 1, **dadurch gekennzeichnet, daß** die Hydrofluoretherkomponente eine perfluorierte Komponente ist, insbesondere der allgemeinen Formel CₙHₘFₚ-O-CₓH_{y}F₂, in der n für eine Zahl zwischen 1 und 12 steht; m für eine Zahl zwischen 0 und 25 steht; p für eine Zahl zwischen 0 und 11 steht; m+p=2n+1; x für eine Zahl zwischen 1 und 12 steht; y für eine Zahl von 0 bis 25 steht; x für eine Zahl von 0 bis 11 steht und y+z=2x+1, wobei in dieser Formel ausgeschlossen ist, daß m und y zugleich gleich 0 sind und daß p und z zugleich gleich 0 sind.

3. Zusammensetzung nach Anspruch 1 oder 2, **dadurch gekennzeichnet, daß** die Hydrofluoretherkomponente ausgewählt ist aus Methoxynonafluorbutan, Ethoxynonafluorbutan, Propoxyundekafluorpentan und Methoxyheptafluorpropan.

4. Zusammensetzung nach einem der vorhergehenden Ansprüche, **dadurch gekennzeichnet, daß** der zuvor genannte Polysäure-Ester ein Polysäure-Ester ist, der hydroxyliert sein kann oder nicht, und der vorzugsweise mit einem gesättigten oder ungesättigten, linearen oder verzweigten Alkohol mit einer Anzahl Kohlenstoffatomen zwischen 1 und 30, vorzugsweise zwischen 1 und 12 gebildet ist, insbesondere Ethanol, Isopropanol oder 2-Ethylhexanol.

5. Zusammensetzung nach einem der vorhergehenden Ansprüche, **dadurch gekennzeichnet, daß** die Polysäure eine Anzahl Kohlenstoffatome zwischen 3 und 10 aufweist und eine lineare oder verzweigte, gesättigte oder ungesättigte Kohlenstoffkette aufweist, wobei die Kohlenstoffkette durch eine oder mehrere Hydroxygruppen substituiert sein kann oder eine oder mehrere Ketonfunktionen umfassen kann und die Hydroxygruppen acetyliert sein können.

6. Zusammensetzung nach einem der vorhergehenden Ansprüche, **dadurch gekennzeichnet, daß** die Polysäure vollständig durch einen Alkohol, wie er in Anspruch 4 definiert ist, verestert ist.

7. Zusammensetzung nach einem der vorhergehenden Ansprüche, **dadurch gekennzeichnet, daß** die Polysäure eine gesättigte zweibasige Säure wie z.B. Malon-, Bernstein-, Glutar-, Adipin-, Pimelin-, Suberin-, Azelainsäure, eine einfach ungesättigte zweibasige Säure wie z.B. Fumar-, Malein-, Zitrakon-, Itakon-, Mesakonsäure, eine zweifach ungesättigte zweibasige Säure wie z.B. Mukonsäure, eine monohydroxylierte zweibasige Säure wie z.B. Tartron-, Apfel-, α-Methylhydroxybutansäure, eine dihydroxylierte zweibasige Säure wie z.B. Dihydroxy-Malein-, Weinsäure, eine tetrahydroxylierte zweibasige Säure wie z.B. Dihydroxy-Wein-, Galactar-, Glukarsäure, eine zweibasige Ketosäure wie z.B. Mesoxal-, Oxal-, 2-Oxoglutar-, 3-Oxoglutarsäure, eine zweibasige Diketosäure wie z.B. 2,3-Diketoadipinsäure, eine gesättigte dreibasige Säure wie z.B. Tricarballyl-, Zitronensäure, oder eine ungesättigte dreibasige Säure wie z.B. Akonitsäure ist.

8. Zusammensetzung nach Anspruch 7, **dadurch gekennzeichnet, daß** die Polysäure Zitronensäure oder Adipinsäure ist.

9. Zusammensetzung nach einem der vorhergehenden Ansprüche, **dadurch gekennzeichnet, daß** der Polysäureester ein im Wesentlichen nicht-polarer Ester ist.

10. Zusammensetzung nach einem der vorhergehenden Ansprüche, **dadurch gekennzeichnet, daß** der vorgenannte Polysäureester ausgewählt ist aus der Gruppe, bestehend aus Triethylcitrat, Tri-(2-ethylhexyl)citrat, Diisopropyladipat und Di-(2-ethylhexyl)adipat.

11. Zusammensetzung nach einem der vorhergehenden Ansprüche, **dadurch gekennzeichnet, daß** der vorgenannte Polysäureester 0,1 bis 30 Gew.-% und insbesondere 1 bis 20 Gew.-% der Hydrofluorkomponente darstellt.

12. Zusammensetzung nach einem der vorhergehenden Ansprüche, **dadurch gekennzeichnet daß** sie eine zusätzliche Komponente enthält, insbesondere ein zweites Co-Lösungsmittel wie z.B. ein Silikon, oder eine Komponente, die die Eigenschaften einer parfümierenden Zusammensetzung wie z.B. die Remanenz der Zusammensetzung auf der Haut verbessert, zum Beispiel ein Phthalat wie z.B. Diethylphthalat.

13. Zusammensetzung nach einem der vorhergehenden Ansprüche, **dadurch gekennzeichnet, daß** sie ein Silikon umfaßt, insbesondere eines ausgewählt unter einem flüchtigen Silikon, einem Dimethikon, einem Cyclomethikon, insbesondere Pentacyclomethikon, oder einem Organotrisiloxan, wobei das Silikon 1 bis 20 Gew.-% der Zusammensetzung darstellt.

14. Verwendung eines Esters einer Polysäure wie er in einem der vorhergehenden Ansprüche definiert ist mit einem Hydrofluorether als Lösungsmittel eines Parfumkonzentrats, insbesondere im Hinblick auf die Zubereitung einer parfümierenden Zusammensetzung wie z.B. Parfums oder Eaux de Toilette.

15. Parfümierende Zusammensetzung ohne Ethanol oder Alkohol, die ein Parfumkonzentrat und einen Hydrofluorether enthält, **dadurch gekennzeichnet, daß** sie außerdem mindestens einen Polysäureester in ausreichender Menge enthält, um ein im Wesentlichen klares Erscheinungsbild der Zusammensetzung zu erhalten.

16. Parfümierende Zusammensetzung nach Anspruch 15, **dadurch gekennzeichnet, daß** die Hydrofluoretherkomponente in einer Menge von ungefähr 65 bis ungefähr 85 Gew.-% in Bezug auf das Endgewicht der parfümierenden Zusammensetzung gegenwärtig ist.

17. Zusammensetzung nach Anspruch 15 oder 16, **dadurch gekennzeichnet, daß** sie ungefähr 1 bis ungefähr 20 Gew.-% Polysäureester, vorzugsweise 3 bis 20 Gew.-% in Bezug auf das Endgewicht der parfümierenden Zusammensetzung enthält.

18. Zusammensetzung nach einem der Ansprüche 15 bis 17, **dadurch gekennzeichnet, daß** sie ungefähr 5 bis ungefähr 20 Gew.-% Parfumkonzentrat enthält.

19. Zusammensetzung nach einem der Ansprüche 15 bis 18, **dadurch gekennzeichnet, daß** es sich um ein Eau de Toilette handelt, das eine Konzentration an Parfumkonzentrat von ungefähr 5 Gew.-% in Bezug auf das Endgewicht des Eau de Toilette enthält.

20. Zusammensetzung nach einem der Ansprüche 15 bis 19, **dadurch gekennzeichnet, daß** es sich um ein Parfum handelt, wobei die Konzentration an Parfumkonzentrat zwischen 10 und 20 Gew.-% in Bezug auf das Endgewicht des Parfums liegt.

21. Zusammensetzung nach einem der Ansprüche 15 bis 20, **dadurch gekennzeichnet, daß** der Hydrofluorether und/oder der Polysäureester so beschaffen ist wie in einem der Ansprüche 1 bis 11 definiert ist.

22. Zusammensetzung nach einem der Ansprüche 15 bis 21, **dadurch gekennzeichnet, daß** sie eine zusätzliche Komponente enthält, insbesondere ein zweites Co-Lösungsmittel wie z.B. Silikon, oder eine Komponente, die die Eigenschaften einer parfümierenden Zusammensetzung verbessert, wie z.B. die Remanenz der Zusammensetzung auf der Haut, z. B. ein Phthalat wie z.B. Diethylphthalat.

23. Zusammensetzung nach einem der Ansprüche 15 bis 22, **dadurch gekennzeichnet, daß** sie bis zu 2 Gew.-% der Endzusammensetzung von mindestens einem zusätzlichen Additiv enthält, z.B. einen UV-Filter, ein oxidationshemmendes Mittel oder einen Farbstoff.

## Claims

1. Ethanol-free or alcohol-free composition comprising a hydrofluoro ether, **characterized in that** it comprises at least one polyacid ester.

2. Composition according to claim 1, **characterized in that** the hydrofluoro ether component is a perfluorinated component, particularly of the general formula CₙHₘFₚ-O-CₓH_{y}F_{z}, in which n is a number ranging from 1 to 12, m is a number ranging from 0 to 25, p is a number ranging from 0 to 11, m + p = 2n + 1, x is a number from 1 to 12, y is a number from 0 to 25, x is a number from 0 to 11 and y + z = 2x + 1, and in which m and y may not be equal to 0 simultaneously and p and z may not be equal to 0 simultaneously.

3. Composition according to claim 1 or 2, **characterized in that** the hydrofluoro ether component is selected from methoxynonafluorobutane, ethoxynonafluorobutane, propoxyundecafluoropentane and methoxyheptafluoropropane.

4. Composition according to one of the preceding claims, **characterized in that** the abovementioned polyacid ester is an ester of a hydroxylated or non-hydroxylated polyacid and is preferably formed with a saturated or unsaturated, linear or branched alcohol having between 1 and 30 carbon atoms, preferably between 1 and 12 carbon atoms, particularly ethanol, isopropanol or 2-ethylhexanol.

5. Composition according to any one of the preceding claims, **characterized in that** the polyacid has between 3 and 10 atoms and comprises a linear or branched, saturated or unsaturated carbon chain, it being possible for said carbon chain to be substituted by one or more hydroxyl groups or to contain one or more ketone groups, and it being possible for said hydroxyl groups to be acetylated.

6. Composition according to any one of the preceding claims, **characterized in that** the polyacid is totally esterified with an alcohol as defined in claim 4.

7. Composition according to any one of the preceding claims, **characterized in that** the polyacid is a saturated diacid such as malonic, succinic, glutaric, adipic, pimelic, suberic or azelaic acid, a monounsaturated diacid such as fumaric, maleic, citraconic, itaconic or mesaconic acid, a diunsaturated diacid such as muconic acid, a monohydroxylated diacid such as tartronic, malic or citramalic acid, a dihydroxylated diacid such as dihydroxymaleic or tartaric acid, a tetrahydroxylated diacid such as dihydroxytartaric, galactaric or glucaric acid, a keto diacid such as mesoxalic, oxalacetic, 2-oxoglutaric or 3-oxoglutaric acid, a diketo diacid such as 2,3-diketoadipic acid, a saturated triacid such as tricarballylic or citric acid, or an unsaturated triacid such as aconitic acid.

8. Composition according to claim 7, **characterized in that** the polyacid is citric acid or adipic acid.

9. Composition according to any one of the preceding claims, **characterized in that** the polyacid ester is a substantially non-polar ester.

10. Composition according to any one of the preceding claims, **characterized in that** the abovementioned polyacid ester is selected from the group comprising triethyl citrate, tri(2-ethylhexyl) citrate, diisopropyl adipate and di(2-ethylhexyl) adipate.

11. Composition according to any one of the preceding claims, **characterized in that** the abovementioned polyacid ester represents 0.1 to 30% by weight, particularly from 1 to 20% by weight, of the hydrofluoro ether component.

12. Composition according to any one of the preceding claims, **characterized in that** it comprises a complementary component, particularly a second co-solvent such as a silicone, or a component for improving the properties of a perfume composition, such as the persistence of said composition on the skin, an example being a phthalate such as diethyl phthalate.

13. Composition according to any one of the preceding claims, **characterized in that** it comprises a silicone selected in particular from a volatile silicone, a dimethicone, a cyclomethicone, particularly pentacyclomethicone, and an organo-trisiloxane, the silicone preferably representing from 1 to 20% by weight of the composition.

14. Use of a polyacid ester as defined in any one of the preceding claims with a hydrofluoro ether as a solubilizer for a perfume concentrate, especially with a view to preparing a perfume composition such as a perfume or toilet water.

15. Ethanol-free or alcohol-free perfume composition comprising a perfume concentrate and a hydrofluoro ether, **characterized in that** it also comprises at least one polyacid ester in a sufficient amount to give said composition an essentially clear appearance.

16. Perfume composition according to claim 15, **characterized in that** the hydrofluoro ether component is present in an amount of about 65 to about 85% by weight, based on the final weight of the perfume composition.

17. Composition according to claim 15 or 16, **characterized in that** it comprises from about 1 to about 20% by weight, preferably from 3 to 20% by weight, of polyacid ester, based on the final weight of the perfume composition.

18. Composition according to any one of claims 15 to 17, **characterized in that** it comprises from about 5 to about 20% by weight of perfume concentrate.

19. Composition according to any one of claims 15 to 18, **characterized in that** it is a toilet water comprising a concentration of perfume concentrate in the order of 5% by weight, based on the final weight of the toilet water.

20. Composition according to any one of claims 15 to 19, **characterized in that** it is a perfume, the concentration of perfume concentrate being between 10 and 20% by weight of the final perfume composition.

21. Composition according to any one of claims 15 to 20, **characterized in that** the hydrofluoro ether and/or the polyacid ester are as defined in any one of claims 1 to 11.

22. Composition according to any one of claims 15 to 21, **characterized in that** it comprises a complementary component, particularly a second co-solvent such as a silicone, or a component for improving the properties of a perfume composition, such as the persistence of said composition on the skin, an example being a phthalate such as diethyl phthalate.

23. Composition according to any one of claims 15 to 22, **characterized in that** it comprises up to 2% by weight, based on the final composition, of at least one complementary additive, for example a UV filter, an antioxidant or a dye.
